(19) Europäisches Patentamt
European Patent Office
Office européen des brevets

(11) Publication number: **0 075 475 A2**

(12) # EUROPEAN PATENT APPLICATION

(21) Application number: 82304944.0

(22) Date of filing: 20.09.82

(51) Int. Cl.³: A 61 K 7/09

(30) Priority: 21.09.81 US 304288

(43) Date of publication of application: 30.03.83 Bulletin 83/13

(84) Designated Contracting States: AT BE CH DE FR GB IT LI LU NL SE

(71) Applicant: SYNTEX (U.S.A.) INC., 3401 Hillview Avenue, Palo Alto, California 94304 (US)

(72) Inventor: Lindenthal, Margaret G., 1417 Village Court, Mt. View California 94040 (US)
Inventor: Edelstein, Herbert, 1178 Hyde Avenue, San Jose California 95129 (US)

(74) Representative: Armitage, Ian Michael et al, MEWBURN ELLIS & CO. 2/3 Cursitor Street, London EC4A 1BQ (GB)

(54) **A composition and process for treating hair.**

(57) An aqueous hair treating composition and a process for its use are disclosed. The composition comprises a water soluble bisulfite salt, a cationic surfactant penetrating agent and a water-soluble organic acid salt buffered to a pH of between 5.0–6.0. The process for treating hair comprises applying said composition to the hair, shaping the hair and, after an appropriate time, rinsing the hair with water.

EP 0 075 475 A2

# A COMPOSITION AND PROCESS FOR TREATING HAIR

Hair waving requires the breaking, rearranging and reforming of keratin protein bonds which are responsible for hair configuration, such as covalent disulfide bonds, ionic bonds between adjacent ionized amino acids of different polarity, and short range polar bonds, e.g. hydrogen bonds. Rearranging ionic and hydrogen bonds can be quickly done with water, wet heat or both in conjunction with mechanical shaping devices. Unfortunately these methods produce only short term results. Permanent hair shaping can best be achieved by breaking, physically rearranging and reforming the cystine disulfide bond (-S-S-) linkages prevalent in keratin protein.

Affecting natural hair shape by lysing the cystine disulfide linkage is referred to as softening or relaxing

the hair. Softening can be done by chemical reduction or with steam. Chemical reduction is usually preferred. Once the hair has been softened, or during the softening step, the hair can be artificially shaped to a desired configuration. The reducing chemicals are then neutralized or washed from the hair, followed generally by an oxidation step to regenerate disulfide linkages between the same or previously unconnected cysteine moieties. Reformation of cystine disulfide bonds or other sulfur-based intrachain protein linkages is sometimes referred to as "hardening." These new cystine disulfide bonds permanently maintain the hair in the shape it was given during the softening and hardening steps.

Hair softening chemicals are generally called hair waving agents, though the same agents may be used to straighten hair as well. At present, one of the most widely used types of hair waving and straightening compositions are the sulfur-based cold wave solutions. Their name is derived from the fact they can provide good wave or straightening action at low (e.g. room) or moderate temperatures, rather than requiring the use of steam or high temperatures during the softening process.

Almost all cold wave solutions contain a reducing agent comprising a sulfur-containing chemical, most commonly thiol substituted acids, aldehydes and alcohols; or bisulfite salts and sulfites. A conventional method for utilizing such solutions is to treat the hair with a solution containing one of these reductive ingredients at alkaline pH, forming the hair into the desired configuration and then treating it with a so-called neutralizing solution or an oxidizing reagent such as a bromate, perborate, or hydrogen peroxide to effect reformation of sulfide bonds. This method enables



BAD ORIGINAL

satisfactory permanent hair shapes to be formed at room temperature.

Among the thiol substituted compounds, thioglycolic acid salts are the most frequently used cold waving agents. However, these particular acid salts or other thiol acid salts, even though they can be used at low temperature, exhibit several problems from the standpoint of hygiene and safety even when used under strictly controlled conditions and in optimized amounts. Waving solutions based on thioglycolic acid frequently emit a strong unpleasant sulfur-based odor due to the presence of mercaptan compounds. This odor is a nuisance to cosmetologists and users. Also, thioglycolic acid salt-based waving solutions only provide good cold wave permanents at a pH of 8.0 or above, which requires the use of an alkaline substance to adjust pH. However, the adjustment of pH, usually done with ammonia, increases the unpleasantness of the sulfur-based odor and the ammonia, or any other base, may cause irritation and sensitization upon contact with the skin.

Alternate room temperature-active hair softening compositions based on the sulfite or bisulfite moiety are known. Sulfite and bisulfite-based solutions exhibit lower acute toxicity and lessened reaction with human skin in relation to thioglycolic acid-based compositions.

An early U.S. patent to J. Speakman, number 2,201,929, discloses the use of sulfites as softening agents in solutions at pHs ranging from 4 through 11, preferably at pH 11. Variations of Speakman's original sulfite and bisulfite cold wave solutions have been proposed. For example, there is a British Patent No. 591,936 to J. Speakman disclosing the addition of 15% to 45% alcohol to such sulfite and bisulfite cold wave lotions. The replacement of alkali sulfites and bisulfites with the sulfites of organic bases, such as



BAD ORIGINAL

monoethanolamine, ethylenediamine, and morpholine are disclosed in U.S. Patent No. 2,437,965 to E. Michaels. In addition, sulfite-based cold wave lotions containing sulfocyanate and diethylene glycol ethers are disclosed in French Patent No. 1,076,766 to J. Scandel; bisulfites and urea and urea derivatives, for example, thiourea, formamide, acetamide, di and triacetamine are disclosed in French Patent No. 1,039,222; and different glycol ethers such as Cellosolve, Carbitol, dioxane, and trioxane in combination with sulfite and bisulfite are disclosed in French Patent No. 1,073,465. These various additives are all acclaimed to intensify the hair setting action of the sulfite/bisulfite combination. Each is most effective at a pH above 6 and requires the use of some user-applied neutralizing or oxidizing chemical other than water as an after-treatment.

Other bisulfite-based hair setting compositions have been proposed, some requiring no special after-treatment other than a water rinse. For example U.S. Patent No. 3,966,903 discloses a hair waving composition which comprises a sulfite and bisulfite waving agent and an alkylene carbonate or alpha or delta lactone accelerating agent, requiring only water rinsing to effect the hair set. These solutions are effective in a pH range from 6 to 9. U.S. Patent 4,038,959 discloses a hair treating composition comprising a bisulfite salt and a mink oil based quaternary ammonium salt conditioning agent. This composition is effective below pH 7, preferably 6.5-6.9. A neutralization step is optional. In another instance, a hair shampoo for increasing hair body is disclosed in U.S. Patent No. 4,243,659. This composition comprises a bisulfite salt, a urea-derived swelling agent, an auxiliary swelling agent which is a polyhydroxy alkyl solvent and a cationic hair conditioner adjusted to a pH between 4.0 to 6.9, preferably 6.0 to 6.6. See also U.S.



BAD ORIGINAL

Patent 3,912,808 and U.S. Patent 2,836,185 which disclose bisulfite reducing agents for treating hair in combination with amine polymers and swelling agents such as water-soluble, nitrogen-containing compounds of the amide class. U.S. Patent 3,227,615 discloses the use of water-soluble bisulfite and a water-soluble cationic polyamide-epichlorohydrin resin for the permanent waving of hair.

A monograph on sulfite cold waving and hair straightening, authored by W. R. Markland, can be found in Norda® Briefs, No. 493, July/August 1979.

All these references disclose systems which include a second reducing chemical and/or recommend the use of a neutralizing or oxidizing agent as an after-treatment in order to stop the reduction reaction and to achieve an acceptable permanent wave. While these systems work well, the ideal and most convenient hair waving system would be one which requires only the application of a reducing composition buffered to the pH of hair, shaping of the hair, application of low to moderate heat, and water rinsing to effectuate the set. It is the object of this invention to provide a hair treating composition which is optimally active at the normal pH of hair, is effective at low to moderate temperatures and does not require the subsequent application of a neutralizing or oxidizing solution.

One aspect of this invention is an aqueous hair treating composition which comprises a water-soluble bisulfite salt, a water-soluble, alkyl-substituted betaine or quaternized amido amine-derived cationic penetrating agent and a water-soluble organic acid salt maintained at a pH between 5.0 and 6.0. The composition optionally includes a buffering agent and an antioxidant.

Another aspect of this invention is a process for treating hair, which process comprises applying the above-described composition to hair for a time sufficient to effect hair treatment with low to moderate heat and then rinsing the hair with water.

There is disclosed herein a hair treating composition which gives unique and surprising hair treating results. The optimum activity of these compositions occurs at the normal pH of hair. Low to moderate temperatures will readily effect the reduction process. No after-treatment other than a water rinse is required to effect permanent hair treatment. Hair may be waved or straightened by applying this composition to hair, shaping the hair either by curling or straightening while applying low to moderate heat, and after an appropriate period of time, rinsing the hair with water. No further treatment is needed or required to impart a suitably long-lasting and manageable permanent shape to the hair.

For the purposes of this invention the phrase "hair treating" should be understood to include curling or waving, straightening, training and any other treatment which gives the hair a desired configuration by means of treatment with the subject compositions in accordance with the process discussed below. "Permanent" refers here to the situation where the hair retains its post-treatment configuration even after a large number of washings or wettings with non-reductive materials.

Water-soluble bisulfite salts are the reducing agents in these unique compositions. They are convenient and safe chemical agents for reducing cystine disulfide linkages and are active at low to moderate temperatures,



BAD ORIGINAL

which in this instance refers to temperatures which vary between about 25° to 50°C. They are essentially odorless and can be formulated into stable systems at an acid pH. In proper concentrations, the subject bisulfite salts do not exhibit dermatological toxicity or sensitization.

The full range of water-soluble bisulfite salts may be used in the practice of this invention. For the purposes of this invention it is most convenient, and therefore preferred, to use a sodium, potassium, ammonium or alkanolamine salts, for example, mono, di or triethanolamine bisulfites. Sodium bisulfite is the most preferred reducing agent herein. Alkali metal metabisulfites, which hydrolyze to bisulfites in water, may also be used though they are not preferred over sodium bisulfite. The anhydrous form of sodium bisulfite usually is supplied as sodium metabisulfite. The instant compositions will contain between 5.0 and 15.0 weight/weight percent of bisulfite salt, preferably in an amount of 10%. These figures apply to all water-soluble bisulfite salts having utility herein regardless of the cation.

The second and a seminal component of this composition is a water-soluble organic acid salt. A "water-soluble organic acid salt" means the salt of a carboxylic acid function-containing, hydrocarbon-derived compound which is sufficiently water-soluble to give a functional concentration and which, at the concentration employed, exhibits no untoward or deleterious dermatological effects.

Such a salt surprisingly has been found to materially and substantially enchance the hair treating activity of the composition. By adding an appropriate amount of such salt, the bisulfite salt-based composition is rendered effective in a pH range lower than previously practiced with bisulfite salt-based hair treating



BAD ORIGINAL

compositions. Further, the presence of this salt reduces treatment time and temperature requirements. Hair texture, feel and strength are also enchanced by addition of this salt. Without this salt, a bisulfite salt or bisulfite salt/cationic penetrating agent hair treating composition does not provide the long lasting permanent or acceptable hair qualities seen when the salt is present.

Water-soluble organic acid salts in general will function in this invention so long as they have the requisite water solubility and are non-toxic. Water solubility will vary depending on the organic acid selected and the counter ion. While there may be instances where one salt form will not demonstrate the requisite solubility, such will not foreclose the use of other salts of that acid. Additionally, while preferred and most preferred concentration ranges are set out infra, a salt need not be water-soluble over the full concentration range in order to come within the scope of this invention. It will be sufficient for this invention if a particular salt is water-soluble at the lower limit of the preferred concentration range.

For the purposes of this invention, water-soluble organic acid salts include the salts of simple organic acids such as the formates, acetates, propionates and the like; di or triacid salts, such as oxalates, malonates, succinates and citrates; mono or polyhydroxy substituted organic acid salts, for example glycolates, lactates, citrates, glucoronates, ascorbates and the like; and aryl acid salts such as benzoates. This is an exemplary list of organic acid salts and it should not be taken as limiting of the organic acid salts which can be used in the practice of this invention. Only water solublity and cosmetological toxicity are limiting herein.



BAD ORIGINAL

The cation may be a metal, an amine, or an alkyl or alkanolamine and the like, so long as the salt has the requisite water solubility. Acid salts will most commonly be the sodium, potassium or ammonium salts. For the practice of this invention it is preferred to use an acetate salt, most preferably sodium acetate. Organic acid salt concentrations may vary between 0.5 and 20.0 weight/weight percent (w/w), most preferably in an amount between 1.0% and 10%.

The composition of this invention also contains a water-soluble, alkyl substituted betaine-based or quaternized amido amine-based surfactant. While numerous cationic surfactants are known to act as penetrating agents, it has been found that only the described compounds are efficacious in combination with the other two seminal components. Anionic, nonionic, amphoteric and like surfactants were found to have little or no effect on the hair treating activity of the water-soluble bisulfite/organic acid salt composition.

The general formula for the alkyl substituted betaine surfactant is:

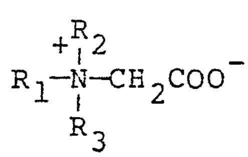

(A)

wherein $R_1$ is a saturated or unsaturated long chain fatty acid radical of 12 to 20 carbon atoms and $R_2$ and $R_3$ represent a short straight or branched chain alkyl or hydroxyalkyl radicals of 1 to 4 carbon atoms. The most preferred surfactant of this type is the one according to Formula A wherein $R_2$ and $R_3$ are both hydroxyethyl and $R_1$ is derived from the fatty acid complex called tallow.

Tallow is defined in the CTFA* Cosmetic Ingredient Dictionary (*Cosmetic, Toiletry and Fragrance



BAD ORIGINAL

Association) as the fat from fatty tissue of sheep or cattle. It consists primarily of fatty acid glycerides. The highest grade of beef tallow is called oleo tallow. It contains (as glycerides): oleic acid (37-43%), palmitic acid (24-32%), stearic acid (20-25%), myristic acid (3-6%), linoleic acid (2-3%) with additional minor constituents such as cholesterol, arachidonic, elaidic, and vaccenic acids.

This preferred surfactant is designated bis-(2-hydroxyethyl) tallow ammonium ethanoate or, by the CTFA Dictionary, dihydroxyethyl tallow glycinate. It is commercially available from Miranol Chemical Co., Inc., South Brunswick, New Jersey, 08810, under the trade name Mirataine TM.

Other examples of alkyl substituted betaine-derived cationic surfactant penetrating agents are lauroamphoglycinate (and) sodium trideceth sulfate (commercially available as Miranol MHT from Miranol Chemical Co., Inc.), cocamidopropyl betaine (commercially available as Tego-betain L-7 from Goldschmidt Chemical Corporation, Hopewell, Va.), cocoamphocarboxyglycinate (commercially available as Miranol C2M from Miranol Chemical Co., Inc.), coco-betaine (commercially available as Standapol AB-45 from Henkel, Inc., Teaneck, N.J.), lauroyl sarcosine (commercially available as Hamposyl-L from W.R. Grace & Co., Organic Chemicals Division, Nashua, N.H.), and cocamidopropyl hydroxysultaine (commercially available as Lonzaine CS from Lonza, Inc., Fairlawn, N.J.).

Alternatively, there may be used a water-soluble quaternary amido amine of Formula B



BAD ORIGINAL

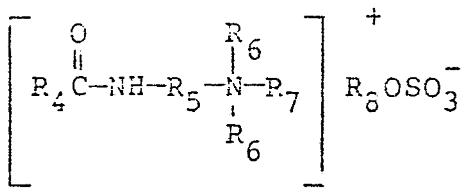

(B)

wherein $R_4$ is a saturated or unsaturated fatty acid hydrocarbon radical of 12 to 20 carbons and $R_5$ through $R_8$ are alkyl radicals of 1 to 4 carbon atoms or hydrogen.

Other examples of water-soluble quaternary amido amine-derived surfactants include laurylpyridinium chloride (commercially available as Dehyquart C from Henkel, Inc.), stearalkonium chloride (commercially available as Ammonyx 4002 from Onyx Chemical Company, Millmaster Onyx Group, Jersey City, N.J.), isostearylethyl imidonium ethosulfate (commercially available as Schercoquat IIS from Scher Chemical Company, Inc.), ricinoleamidopropyl ethyldimonium ethosulfate (commercially available as Lipoquat R from Lipo Chemicals, Inc., Patterson, N.J.), quaternium 27 or tallowamido ethylimidazomonium methosulfate (commercially available as Carsosoft S-75 from Lonza, Inc.), and olealkonium chloride (commercially available as Alacsan 7LUF from Alcolac, Inc., Baltimore, Md.).

Most preferred is the compound according to Formula B wherein $R_4$ is the isostearyl fatty acid radical, $R_5$ is propyl, $R_6$ is methyl and $R_7$ and $R_8$ are ethyl, being the compound N,N-dimethyl-N-ethyl-N-(3-isostearyl) amidopropyl ammonium ethyl sulfate. Its CTFA Dictionary designation is isostearamidopropyl ethyldimonium ethosulfate. Such material is available from the Scher Chemical Co., Inc., Clifton, New Jersey, 07012, under the trade name Schercoquat IAS-LC.

These penetrating agents may be used singly or in combination in amounts between 0.1 and 3.0 weight/weight



BAD ORIGINAL

percent (w/w). Preferably the total amount of penetrating agent will be 0.25 to 1.5% (w/w).

It has surprisingly been determined that cationic surfactant penetrating agents having a molecular weight below about 1000 are best for facilitating penetration of the cuticle and retention in the hair shaft with the compositions of the present invention.

These compositions are adjusted to a pH of between 5 and 6, preferably between 5.3 - 5.5. It is important to maintain the pH of these compositions within a pH of 5 to 6 for several reasons. First, this pH range maximizes the reducing capacity of the composition; secondly, this pH range stabilizes the composition; and thirdly, the normal pH of hair falls in this range.

Bisulfite ion is the most effective cystine disulfide bond reducing agent vis-a-vis the sulfite ion. Below pH 6 bisulfite ion is the only species present while above that pH the solution represents an equilibrium between bisulfite and sulfite ions.

Stability considerations indicate it is desirable to maintain this pH range. Below pH 5, this tripartite composition is unstable. After several days at room temperature it gives off noxious fumes and shows a concurrent decrease in activity. However, adjusting the pH to above 5.0 greatly improves composition stability and long term efficacy.

Since the normal pH range of hair is 5.3 - 5.5, it is most desirable to treat hair at this pH in order to minimize adverse affects to the hair. This pH range also is less likely to sensitize or irritate the scalp or other skin which may come in contact with the composition.

The instant aqueous solutions will normally have an acid pH, usually within or very close to the preferred range. However, it is best to monitor the pH during the



BAD ORIGINAL

formulation process and make an appropriate adjustment with acid or base as the last formulation step.

If pH adjustment is needed, a dilute solution of acid or base is best used. Any composition-compatible acid or base may be used for this purpose. If the pH must be lowered it is preferred to use the acid form of the organic acid salt for this purpose. Where the pH must be adjusted upward, the most frequent case, ammonium hydroxide or an alkanolamine are the preferred alkali materials. Other nitrogen-derived bases, alkaline earth metal hydroxides and the like will serve this purpose equally well. It is the adjustment of pH which is of significance here, not the particular acid or alkali used.

Although not required, it is preferable to include a buffering agent in these compositions to stabilize the pH within the preferred range. Such materials, weak acids and their salts, must have some buffering capacity in the preferred pH range and be compatible with the other composition materials. Depending on the chosen water-soluble organic acid salt, that salt can be incorporated into the composition as one of the buffering agent components. An acetate salt-acetic acid buffer system is exemplified hereinafter, and is the preferred buffering agent for this invention where such is employed. Other weakly acidic systems with some buffering capacity in the preferred pH range will function equally well. The total percentage of buffering material, when present, should be in the range of 0.01% to 2.0% by weight/weight of the total composition. Where the buffer system is an organic acid/salt combination wherein the salt is first an active ingredient as discussed above, the amount of acid will be present in accordance with the 0.01% to 2.0% range, but the amount of salt present will conform to the functional range



BAD ORIGINAL

noted above for an organic acid salt as an active ingredient.

Antioxidant reducing agents may be added to these compositions if desired. They will serve to improve the composition's stability and can indirectly assist in maintaining pH. Any suitable compatible antioxidant useful in pharmaceutical or cosmetological compositions can be used. Erythrobic acid and ascorbic acid, for example, can be used for this purpose. An antioxidant will be present in an amount of about 0.1 to 0.3 weight/weight percent.

Further, if it is desired, perfumes and the like may be added to these compositions so long as they do not adversely affect the hair treating activity of the composition.

The process for treating hair according to this invention comprises applying the subject compositions to hair by some convenient means, shaping the hair and resaturating it with solution if desired, followed by the application of low to moderate heat for some time sufficient to effect hair setting. Following these steps, a water rinse will complete the treatment process.

The amount of solution required to effect hair treatment is not exact. However the hair should be well wetted, if not saturated, in order to realize to the fullest extent the hair treating activity of these compositions. If the hair is not fully wetted, the disulfide bond reduction will not be maximized, which can result in less stable permanents.

These compositions can be applied to dry or wet hair, but it is preferred to first wash the hair and then apply the instant compositions while the hair is still wet. The compositions can be applied as a spray, as droplets, or placed on some mechanical device such as a comb and applied to the hair thereby. Generally a more

even distribution of the composition can be achieved by applying the composition to a comb and combing it into the hair, or by combing the hair after the composition has been applied as a spray or droplets.

While it is usual to apply such solutions before shaping the hair, the solution may be reapplied once the hair is on mandrels, in order to insure complete and adequate wetting. After the hair has been wetted and shaped, a plastic bag or some other non-porous material should be placed over the hair to keep it moist during the treatment period.

Heat is not required to effect a good permanent wave with these compositions, but the use of moderate temperatures will expedite the process. A preferred practice of this invention will include the application of some heat to the hair after it has been wetted. Temperatures should not exceed 50°C. A temperature less than this is more than adequate to insure proper and complete hair treatment. Preferably, a temperature of 40° to 45°C will be used.

The amount of time for effecting hair treatment is that time which is sufficient to effect a long lasting permanent hair set. Because the proper amount of time will depend on the hair type, condition of the hair, the degree of hair wetting with the subject compositions and the processing temperature, only a general time range can be specified. The exact time will be determined by the user based on his or her skill and knowledge of the several noted factors.

It has been found that, generally, the hair treating process will not require more than 60 minutes and usually is completed in 15 to 30 minutes, especially when moderate heat is applied. However it should be understood that because hair treatment is dependent on a



BAD ORIGINAL

variety of factors, for a given head of hair, a good permanent may be realized in less than 15 minutes.

Following the reduction step, hair need only be rinsed with running water and dried to effect a set to the hair. Usually the hair will be rinsed while still on mandrels or other shaping device. Several minutes of thorough water rinsing, about 5 minutes or so, generally removes all residual bisulfite/organic acid/surfactant material. The hair can then be removed from the shaping device and dried or, for a more curly hair shape, dried on the shaping devices, at the choice of the user.

The following examples further illustrate the present invention.

## EXAMPLE I

A hair treating composition was prepared by conventional procedures, comprising:

TABLE I

| Ingredient | Amounts |
|---|---|
| Sodium bisulfite | 10.0g |
| Isostearamidopropyl ethyldimonium ethosulfate | 0.5g |
| Acetate buffer* | 20.0ml |
| Ascorbic acid | 0.5g |
| Deionized water | q.s. 100 g |

*Acetate buffer:

| | |
|---|---|
| Sodium acetate | 220g |
| Glacial acetic acid | 20ml |
| Deionized water | q.s. 1 liter |



BAD ORIGINAL

Formulation was carried out by first dissolving the cationic surfactant in 90% of the deionized water with stirring. Next was added the sodium acetate and antioxidant with stirring, followed by the sodium bisulfite. After all materials were dissolved, the pH was adjusted to 5.3 with 10% ammonium hydroxide, after which water is added in a quantity sufficient (q.s.) to bring the composition to total weight (100 g).

EXAMPLE II

This Example factor illustrates the hair treating process of the present invention. Natural brown hair was given a permanent wave using the composition of Example I as follows: Hair was shampooed once with a mild acidic cleansing shampoo, rinsed thoroughly and toweled dry. While the hair may be slightly wet or dry, the moisture content should be even. Solution was applied directly to one section of the hair at a time with combing through for even distribution. Small, thoroughly saturated sections of hair were then wound on mandrels. After all sections had been wound, each section was resaturated with solution. The hair was covered with a plastic bag and air preheated to between 40° and 50°C was applied. Processing required between 15 and 30 minutes. After processing, the hair was rinsed with warm running water while still on the mandrels for at least 5 minutes. A towel was used to blot dry the hair and the hair allowed to rest several minutes before the rods were removed to give a natural looking soft wave style.

A curly hair style can be obtained by drying the hair with heat before removing the rods. Alternatively, hair may be set on rollers in a conventional manner after rod removal.



BAD ORIGINAL

## EXAMPLE III

This example further illustrates the hair treating compositions of this invention.

TABLE II

| Ingredient | Amounts |
|---|---|
| Sodium bisulfite | 10.0g |
| Isostearamidopropyl ethyldimonium ethosulfate | 0.5g |
| Dihydroxyethyl tallow glycinate | 0.5g |
| Acetate buffer* | 10.0ml |
| Erythorbic acid | 0.5g |
| Deionized water | q.s. 100 g |

*Acetate buffer:

| | |
|---|---|
| Sodium acetate | 220g |
| Glacial acetic acid | 20ml |
| Deionized water | q.s. 1 liter |

The ingredients in Table II were combined in the manner noted in Example I, ammonium hydroxide (10%) being used to adjust the pH to 5.3.

## EXAMPLE IV

This example further illustrates the hair treating composition within the teachings of this invention.



BAD ORIGINAL

TABLE III

| Ingredient | Amounts |
|---|---|
| Sodium bisulfite | 10.0g |
| Ammonium hydroxide (10%) | 1.0g |
| Isastearamidopropyl ethyldimonium ethosulfate | 0.5g |
| Ammonium acetate | 1.5g |
| Deionized water | q.s. to 100 g |

Ammonium hydroxide was added by drops (about 18) to the sodium bisulfite, first bringing the pH to 5.35, and second, forming ammonium bisulfite salt. The penetrating agent and ammonium acetate were added with stirring, and deionized water was added to bring the total weight to 100 g.

EXAMPLE V

This example further illustrates a hair treating composition within the teachings of this invention.

TABLE IV

| Ingredient | Amounts |
|---|---|
| Sodium bisulfite | 10.0g |
| Ethanolamine | 9.0g |
| Sodium trideceth - 7 carboxylate | 1.0g |
| Sodium acetate | 1.0g |
| Deionized water | q.s. to 100 g |

The ingredients were mixed together and the pH was adjusted to 5.5 by the addition of phosphoric acid, which acts as a phosphate/acetate buffer system when combined



BAD ORIGINAL

with the sodium acetate. The particular penetrating agent used was minimally compatible with the system, giving a somewhat cloudy solution. Good wave characteristics were obtained.

EXAMPLE VI

This Example further illustrates a hair treating composition within the teachings of this invention.

TABLE V

| Ingredient | Amounts |
|---|---|
| Sodium bisulfite | 10.0g |
| Dihydroxyethyl tallow glycinate | 0.5g |
| Sodium acetate | 1.0g |
| Deionized water | q.s. to 100 g |

This pH 5.0 solution gave very firm curl formation when applied to hair, even after a shampoo 48 hours post treatment.

EXAMPLE VII

This Example further illustrates a hair treating composition within the teachings of this invention.

TABLE VI

| Ingredient | Amounts |
|---|---|
| Sodium bisulfite | 10.0g |
| Ammonium hydroxide (10%) | 0.5g |
| Dihydroxyethyl tallow glycinate | 0.5g |
| Ammonium acetate | 1.0g |
| Deionized water | q.s. to 100 g |

This pH 5.3 solution gave excellent curl formation when applied to hair, even after a shampoo 48 hours post treatment.

EXAMPLE VIII

This Example further illustrates a hair treating composition within the teachings of this invention.

TABLE VII

| Ingredient | Amounts |
|---|---|
| Sodium bisulfite | 10.0g |
| Stearalkonium chloride | 0.5g |
| Sodium acetate | 1.0g |
| Deionized water | q.s. to 100 g |

The pH of this solution was 4.9. It gave an acceptable curl when applied, but, the solution was somewhat cloudy.

EXAMPLE IX

This Example further illustrates a hair treating composition within the teachings of this invention.

TABLE VIII

| Ingredient | Amounts |
| --- | --- |
| Sodium bisulfite | 10.0g |
| Laurylpyridium chloride | 0.5g |
| Sodium acetate | 1.0g |
| Deionized water | q.s. to 100 g |

Laurylpyridium chloride was dissolved in deionized water; then the remaining ingredients were added and the pH was adjusted to 5.1 by the addition of ethanolamine.

EXAMPLE X

This Example further illustrates a hair treating composition within the teachings of this invention.

TABLE IX

| Ingredient | Amounts |
| --- | --- |
| Sodium bisulfite | 10.0g |
| Isostearamidopropyl ethyldimonium ethosulfate | 0.5g |
| Dihydroxyethyl tallow glycinate | 0.5g |
| Acetate buffer* | 5.0 ml |
| Ammonium hydroxide | 1.25g |
| Ascorbic acid | 0.5g |
| Deionized water | q.s. to 100 g |

*See Examples 1 and 3

The above ingredients were combined, adding the ammonium hydroxide by drops (26 drops) to reach a pH of 5.35.



BAD ORIGINAL

EXAMPLE XI

This Example illustrates a presently preferred hair treating composition of this invention.

TABLE X

| Ingredient | Amounts |
|---|---|
| Sodium bisulfite | 10.0g |
| Isostearamidopropyl ethyldimonium ethosulfate | 0.5g |
| Acetate buffer* | 10.0g |
| Erythrobic acid | 0.5g |
| Ammonium hydroxide (10%) to pH 5.3 | about 1.4g |
| Deionized water | q.s. to 100 g |

*See Examples 1 and 3

EXAMPLE XII

This example further illustrates the hair treating composition within the teachings of this invention.

TABLE XI

| Ingredient | Amounts |
|---|---|
| Sodium bisulfite | 10.0 g |
| Cocamidopropyl betaine | 3.15 g* |
| Acetate buffer** | 10.0 g |
| Erythrobic acid | 0.5 g |
| Ammonium Hydroxide (10%) | 1.5 g |
| Deionized water | q.s. 100 g |

*Effective as 1% active in solution.

**See examples 1 and 3.

The above ingredients were combined as described above, resulting in a clear solution which gave good curl after 15 minutes processing time with moderate heat.



BAD ORIGINAL

WHAT IS CLAIMED IS:

1. A hair treating composition requiring only water rinsing as a final chemical treatment to effect complete treatment of the hair, which comprises:

a) a water-soluble bisulfite salt;

b) a water-soluble cationic surfactant penetrating agent; and

c) a water-soluble organic acid salt;

said composition having a pH between 5 and 6.

2. A composition according to Claim 1 which contains:

a) said bisulfite salt in an amount between 5.0 and 15.0 by weight percent (w/w);

b) said penetrating agent in an amount between 0.10 and 3.0% (w/w);

c) said organic acid salt in an amount between 0.5 and 20.0% (w/w); and

d) water in a quantity sufficient to make 100% (w/w).

3. A composition according to Claim 1 wherein said water-soluble cationic surfactant penetrating agent has a molecular weight below about 1000.

4. A composition according to Claims 1, 2 or 3 wherein said water-soluble cationic surfactant penetrating agent is selected from the group consisting of:

alkyl-substituted betaine-derived penetrating agents and quaternized amido amine-derived penetrating agents.



BAD ORIGINAL

5. A composition according to Claim 4 wherein:

a) said bisulfite salt is sodium, potassium, ethanolamine or ammonium bisulfite;

b) said penetrating agent is an alkyl substituted betaine-derived surfactant of the formula

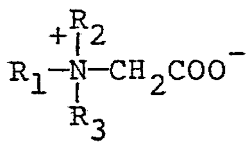

wherein $R_1$ is a saturated or unsaturated long chain fatty acid radical of 12 to 20 carbon atoms and $R_2$ and $R_3$ represent a short straight or branched chain alkyl or hydroxyalkyl radical of 1 to 4 carbon atoms; and

c) said organic acid salt is an ammonium or alkali metal acetate;

said composition being maintained at a pH between 5.3 and 5.5.

6. A composition according to Claim 5 wherein:

a) said bisulfite salt is sodium bisulfite in an amount of 10% (w/w);

b) said penetrating agent is bis-(2-hydroxyethyl) tallow ammonium ethanoate in an amount of 0.25 to 1.5% (w/w);

c) said organic acid salt is sodium acetate in an amount of 1.0 to 10.0% (w/w); and

d) water in a quantity sufficient to make 100% (w/w).

7. A composition according to Claim 4 wherein:

a) said bisulfite salt is sodium, potassium, ethanolamine or ammonium bisulfite;

b) said penetrating agent is an quaternary amido amine-derived surfactant of the formula

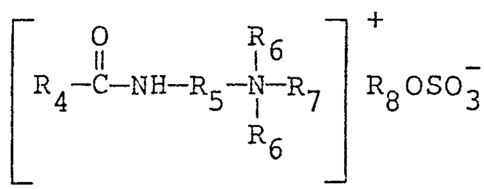

wherein $R_4$ is a saturated or unsaturated fatty acid hydrocarbon radical of 12 to 20 carbons and $R_5$ through $R_8$ are alkyl radicals of 1 to 4 carbon atoms or hydrogen; and

c) said organic acid salt is an ammonium or alkali metal acetate;

said composition being maintained at a pH between 5.3 and 5.5.

8. A composition according to Claim 7 wherein:

a) said bisulfite salt is sodium bisulfite in an amount of 10% (w/w);

b) said penetrating agent is N,N-dimethyl-N-ethyl-N-(3-isostearyl)amidopropyl ammonium ethyl sulfate in an amount of 0.25 to 1.5% (w/w);

c) said organic acid salt is sodium acetate in an amount of 1.0 to 10.0% (w/w); and

d) water in a quantity sufficient to make 100% (w/w).

9. A composition according to Claim 1 including:

a) optionally a buffering agent; and

b) optionally an antioxidant.

10. A composition according to Claim 9 where said buffering agent is a sodium acetate/acetic acid mixture.

11. A process for treating hair, which process comprises:

A. applying to the hair an aqueous composition according to any one of the preceding claims;

B. shaping the hair;

C. applying low or moderate heat for a time sufficient to effect hair treatment; and

D. rinsing the hair with water, said water rinsing being the only chemical treatment after step A required to effect complete treatment of the hair.